# EUROPEAN PATENT SPECIFICATION

(11) **EP 2 018 207 B1**
(45) Date of publication and mention of the grant of the patent: **20.10.2010**
(21) Application number: 07736330.7
(22) Date of filing: 14.05.2007
(51) Int. Cl.: A61M 25/10, A61M 25/01

(54) **BALLOON CATHETER**
BALLONKATHETER
CATHÉTER À BALLONNET

(30) Priority: 15.05.2006 US 800035 P; 12.10.2006 US 546571
(43) Date of publication of application: 28.01.2009
(60) Divisional application: 10151352.1
(73) Proprietor: Endocross Ltd., 20692 Yoqneam (IL)
(72) Inventor: HIRSZOWICZ, Eran, 52236 Ramat-gan (IL); LEVIT, Eran, 25147 Kfar Vradim (IL); DUBI, Shay, Tel-aviv (IL)
(74) Representative: Turner, Craig Robert
(86) International application number: PCT/IL2007/000590
(87) International publication number: WO 2007/132464

(56) References cited:
- EP-A1- 0 540 858
- US-A- 4 781 681
- US-A- 5 318 532
- US-A- 5 733 260
- US-A1- 2005 038 382

## Description

### Field of the Invention

The present invention relates to a balloon catheter device. More particularly, the invention relates to a balloon catheter device for treating occluded body passages.

### Background of the Invention

The present invention is directed to a balloon catheter device that is adapted to be inserted into inner body organs and/or passages, and for in vivo treating obstructions formed therein.

For example, in events of chronic total occlusion of blood vessels, there is complete (or almost complete) obstruction of the vessel due to the development of an intravascular lesion comprising atheromatous plaque material and/or thrombic material. Between 10 and 20 percent of patients undergoing percutaneous coronary interventions (PCI) have CTO (chronic total occlusion). Successful opening of CTO lesions improves anginal status, increases exercise capacity, and reduces the need for bypass surgery. However, PCI of cases of CTO have historically posed problems, with lower- success rates (40 to 80 percent - average 60 percent), higher equipment costs, and a higher restenosis rate.

US 2005/038382 discloses a balloon catheter comprising an inner tube, a medical balloon and an inflation lumen.

US 5318532 discloses a balloon dilatation catheter which can be of the multilumen type with variable cross-section lumens.

EP 540858 describes an intravascular catheter having a catheter shaft with an expandable tubular element on its distal end which upon inflation to an internal pressure at or above a threshold pressure expands in a manner which is related to the internal pressure.

US 5733260 discloses a dilation balloon catheter with a dilation balloon lumen, a guidewire lumen, and an inner balloon lumen.

Conventional intervention tools such as angioplasty balloons are often too flexible or blunt to cross the CTO site, which often contains extremely hard, calcified tissue that may form an impenetrable barrier to the advancement of a guidewire therethrough. Even a less than total occlusion may contain complex structures which may trap or divert the steering end of the guidewire. In view of the great difficulties encountered in attempting to properly position a guidewire across the stenosis, conventional guided atherectomy or dilatation devices such as cutting elements and balloons cannot be used to cross the lesion as long as a guidewire was not inserted through the lesion since they rely on complete wire crossability.

A further problem associated with the use of conventional devices is the risk of perforating the blood vessel being treated. For example, a guidewire or cutting tool, when advanced, may cause dissection of the tissues of the arterial wall instead of the occlusion, thereby creating a false lumen and possibly perforating the artery. If enough blood from a perforated artery accumulates in the pericardial space surrounding the heart, it will result in a condition known as cardiac tamponade in which the heart is compressed and emergency surgical intervention is required to avert heart failure and death.

Another reason that conventional types of apparatuses are typically ineffective in treating total or near total occlusions is that conventional balloon catheter shafts and guidewires do not perform well under the compressive loading and torque loading that are required in order to advance such devices across a CTO lesion.

Statistically, the predominant reason for failure to open CTO lesions with PCI has been failure to cross the lesion with a guidewire (80 percent) and failure of a balloon to track along the guidewire (15 percent) through the very hard lesion. Many types of guidewires and devices have been tried, but successful recanalization has remained at about 60 percent. Crossing CTO lesions in,patients with peripheral vascular disease has met with similar problems, the reported success rate for percutaneous catheter-based treatment of chronic subclavian artery occlusion being in the range of 46%-83%.

It is therefore an object of the present invention to provide a balloon catheter system that is capable of in vivo penetrating and crossing obstructions in body passages.

It is another object of the present invention to provide a system for in vivo penetrating and crossing body obstructions without impairing adjacent tissues and/or body passages.

It is a further object of the present invention to provide a system for penetrating and crossing body obstructions by means of a guidewire having enhanced pushability and centered tip inside the pathway.

Other objects and advantages of the invention will become apparent as the description proceeds.

### Summary of the invention

It has now been found by the inventors that it is possible to achieve the above-mentioned objectives by means of the use of a balloon catheter system fitted with a balloon that is terminally infolded (or a balloon which may be caused to adopt such an intussuscepted configuration). Following delivery of the balloon to the proximal side of the occluding vascular lesion to be treated, said balloon is inflated such that it becomes anchored within the blood vessel. The balloon is then caused to rapidly inflate and partially deflate in a cyclic manner, such that said balloon alternately elongates and axially contracts. The distal portion of the catheter shaft upon which the balloon is mounted, and a guide wire firmly grasped within the lumen of said shaft, and which projects beyond the distal end thereof, is thereby caused to similarly oscillate along a distal-proximal axis (wherein proximal is defined as the direction towards the operator and distal as the direction away from the operator). This longitudinal oscillation of the guide wire in proximity to the occluding lesion to be treated causes damage and break-up of the lesion, ultimately enabling the operator to advance the balloon catheter and/or other conventional angioplasty devices across the lesion.

During the course of their work, the inventors further found that in order to facilitate oscillation of the catheter shaft along a distal-proximal axis in response to the similar elongation-contraction of the balloon, it is desirable that said catheter shaft contains at least one portion that is elastically deformable along said distal-proximal axis. Without wishing to be bound by theory, it is believed that in response to increased pressure inside the proximally intussuscepted balloon, the folded distal balloon tapers are extended in a distal direction thereby exerting tension forces along the catheter shaft. Due to the elasticity of the shaft, the induced tension forces result in elongation of the said shaft, thereby stressing the shaft, similar to a tension spring charging procedure. Partial pressure release inside the balloon reduces these tension forces. The catheter shaft then acts as a returning spring and thereby assists in rolling the distal balloon tapers back to their initial position.

A key feature of the devices of the present invention is the presence of means for grasping and immobilizing a guidewire placed within a guidewire lumen, such that the distal tip of said guidewire extends beyond the distal extremity of the guidewire lumen. The guidewire grasping and immobilizing means is provided by an elastically deformable region of the wall of the guidewire conduit. This elastically deformable region is distinct from the elastically deformable region described hereinabove, the purpose of which is to permit alternate elongation and axial contraction of the inner shaft in response to cyclic inflation/partial deflation of the balloon. It is to be emphasized that in this group of embodiments, the distal portion of the guidewire conduit contains at least two distinct elastically-deformable regions. The first of these regions responds to elevation of the fluid pressure within the catheter to a first pressure increment by means of reducing its diameter, thereby applying a 'strangling' force on the guidewire. The second of these regions responds to further elevation of the fluid pressure by a second, higher pressure increment (and associated elongation of the balloon) by means of axial elongation, and subsequent contraction upon reduction of the pressure by an amount equal to the second pressure increment. The catheter devices in this group of embodiments may further comprise supplementary guidewire grasping elements, as will be disclosed and described hereinafter.

The present invention is thus primarily directed to a balloon catheter comprising:
an inner shaft disposed within an outer shaft such that the distal end of the inner shaft extends beyond the distal end of the outer shaft, wherein the lumen of said inner shaft is suitable for allowing the passage of a guidewire through all or part of its length;
a balloon attached at its proximal end to said outer shaft and at its distal end to said inner shaft; and
means for the introduction of an expansion fluid into the annular space formed between the inner surface of the outer shaft and the outer surface of the inner shaft and therefrom into the lumen of said balloon, and for the removal thereof;
   wherein the inner shaft is constructed such that following radial expansion of the balloon to a first expanded state, a portion of said inner shaft is capable of responding to further longitudinal expansion of the balloon to a second expanded state by reducing its inner diameter thereby being able to grasp a guidewire placed within the lumen of said inner shaft, thereby preventing movement of said guidewire relative to said inner shaft, and such that
said inner shaft is capable of responding to further expansion of the balloon from said second expanded state to a third expanded state by increasing its length from a resting value, and of responding to subsequent partial deflation back to said second expanded state by reducing its length back to said resting value.

In one preferred embodiment, the inner shaft comprises at least a first elastically deformable portion and second elastically deformable portion, wherein, following radial expansion of the balloon to a first expanded state,
said first elastically deformable portion of the shaft is capable of responding to further longitudinal expansion of the balloon to a second expanded state by reducing its inner diameter thereby being able to grasp a guidewire placed within the lumen of said inner shaft and thereby preventing movement of said guidewire relative to said inner shaft,
and wherein said second elastically deformable portion of the shaft is capable of responding to further longitudinal expansion of the balloon from said second expanded state to a third expanded state by increasing its length from a resting value, and of responding to subsequent partial deflation back to said second expanded state by reducing its length back to said resting value.

In one preferred embodiment, the distance between the points of attachment of the balloon to the inner and outer shafts is less than the overall length of the balloon when said balloon is in the first expanded state.

In another particularly preferred embodiment, the first and second elastically deformable portions are each constructed of blends of nylon and Pebax.

In a further preferred embodiment of the invention, the balloon catheter further comprises supplementary guide wire grasping means. It is possible to use any suitable mechanical elements for implementing these supplementary means. In one preferred embodiment, however, the supplementary guidewire grasping means comprise protrusions on the inner wall of the inner shaft. In another preferred embodiment, the supplementary guide wire grasping means comprise wedge-shaped locking elements.

Various balloon attachment configurations may be used in implementing this group of embodiments of the invention. Thus, in one preferred embodiment, the balloon is attached at its distal end in an inverted manner, such that the external surface of said balloon is in contact with, and attached to, the outer surface of the inner shaft. In another preferred embodiment, the balloon is, attached at its distal end in a non-inverted manner, such that the internal surface of said balloon is in contact with, and attached to, the outer surface of the inner shaft.

The presently-disclosed embodiments of the balloon catheter of the present invention may be constructed either in the form of an over-the-wire catheter or as a rapid exchange (single operator) catheter.

In one preferred embodiment of the catheter of the present invention, the inner shaft is capable of being moved along its longitudinal axis in relation to the outer shaft, said catheter further comprising means for immobilizing said inner shaft.

An example further provides a balloon catheter comprising a conduit having proximal and distal portions,
wherein said proximal portion contains two separate lumens, one of said lumens being suitable for allowing the passage of a guidewire therethrough, the other lumen being suitable for allowing passage of a fluid,
wherein said distal portion contains a single lumen, said lumen being the continuation of the guidewire lumen,
wherein a balloon is attached at its proximal end to said proximal conduit portion and at its distal end to said distal conduit portion;
and wherein the distal portion of the conduit is constructed such that following radial expansion of the balloon to a first expanded state, a portion of said distal conduit portion is capable of responding to further longitudinal expansion of the balloon to a second expanded state by reducing its inner diameter thereby being able to grasp a guidewire placed within the lumen of said distal conduit portion, thereby preventing movement of said guidewire relative to said inner shaft, and such that
said distal conduit portion is capable of responding to further expansion of the balloon from said second expanded state to a third expanded state by increasing its length from a resting value, and of responding to subsequent partial deflation back to said second expanded state by reducing its length back to said resting value.

Another example also provides a balloon catheter comprising a conduit having a proximal portion, a middle portion and a distal portion,
wherein said proximal portion contains two separate lumens, one of said lumens being suitable for allowing the passage of a guidewire therethrough, the other lumen, being suitable for allowing passage of a fluid,
wherein said middle portion contains two concentrically arranged conduits, the lumen of the outer conduit being in fluid communication with the fluid passage lumen of said proximal portion, the lumen of the inner conduit being in fluid communication with the guidewire lumen of said proximal portion,
wherein said distal portion is a single-lumen conduit that is in fluid communication with the inner lumen of said middle portion,
wherein a balloon is attached at its proximal end to the outer surface of said middle portion and at its distal end to said distal portion;
and wherein at least part of the conduit of said distal portion is constructed such that following radial expansion of the balloon to a first expanded state, said distal portion conduit is capable of responding to further expansion of the balloon to a second expanded state by reducing its inner diameter thereby being able to grasp a guidewire placed within the lumen of said distal conduit portion, thereby preventing movement of said guidewire relative to said inner shaft, and such that
said middle portion inner conduit is capable of responding to further expansion of the balloon from said second expanded state to a third expanded state by increasing its length from a resting value, and of responding to subsequent partial deflation back to said second expanded state by reducing its length back to said resting value.

In the case of both of the bi-lumen implementations described immediately hereinabove, the inner conduit preferably comprises at least one portion constructed of at least two different shaped elastically deformable segments, each of said segments responding to different fluid pressures.

In one particularly preferred embodiment, the at least two elastically deformable portions comprise various blends of nylon and/or Pebax.

In another example a method for treating vascular occlusions in a patient in need of such treatment, comprising the steps of:
a) bringing a balloon into proximity with a vascular occlusion within a blood vessel to be treated;
b) causing said balloon to be cyclically inflated and partially deflated such that a guidewire attached to said balloon is caused to oscillate along a distal-proximal axis in close proximity to the vascular occlusion, thereby causing complete or partial fracture of said occlusion.

In a particularly preferred example of the above-defined method of treatment, the guidewire is immobilized within a catheter shaft.

Preferably, prior to the cyclical inflation and partial deflation of the balloon, said balloon is inflated to a first pressure, such that said balloon becomes anchored within the blood vessel being treated, and such that during the step of cyclical inflation and partial deflation, the balloon is further inflated by increasing its internal pressure from said first pressure to a second pressure, and partially deflated by reducing its internal pressure from said second pressure to said first pressure.

In one implementation of the examplary method subsequent to anchoring of the balloon within the blood vessel, said method further comprises the step of moving the inner catheter shaft in a proximal direction such that the distal extremity of the balloon becomes intussuscepted. This particular example of a method is generally used in conjunction with balloons that are manufactured such that they do not inherently possess an intussuscepted portion ("non-charged" balloons). The proximal movement of the inner shaft, as mentioned above, thereby causes the formation of an intussusception in the previously non-intussuscepted balloon.

All the above and other characteristics and advantages of the present invention will be further understood from the following illustrative and non-limitative examples of preferred embodiments thereof.

### Brief Description of the Drawings

The present invention is illustrated by way of example in the figures of the accompanying drawings, in which like references indicate similar elements and in which:
- Figs. 1A to 1C demonstrate a preferred embodiment of the invention and a procedure for catheter deployment at the occlusion site;
- Figs. 2A and 2B demonstrate another possible procedure for catheter deployment at the occlusion site;
- Figs. 3A and 3B are longitudinal and cross-sectional views illustrating a balloon catheter device of the invention that is capable of delivering rapid motion to its distal end portion and/or to a treatment tool passing therein;
- Figs. 4A and 4B are longitudinal and cross-sectional views illustrating the balloon catheter device of the invention ramming an occlusion;
- Fig. 5 schematically illustrate the balloon catheter device of the invention comprising coupling means between the inner tube and the ramming tool;
- Fig. 6 schematically illustrates another possible coupling means between the inner tube and the ramming tool; and
- Fig. 7 schematically illustrates a balloon catheter of the invention wherein the inflatable member comprises a narrow distal portion;
- Figs. 8A to 8C illustrate three different examples of the balloon catheter using a bi-lumen conduit instead of a concentric inner tube-outer tube configuration proximal to the balloon attachment;
- Figs. 9A and 9B illustrate two alternative embodiments that may be used when there is a need to maneuver the balloon catheter through a curved region of the vasculature.

### Detailed Description of Preferred Embodiments

The present invention is directed to a balloon catheter system comprising a guidewire (also referred to herein as a ramming tool) immobilized within an inner catheter shaft lumen, wherein the balloon catheter is capable of delivering rapid motion to the guidewire passing therein. The in vivo application of such rapid motion to, or adjacent to, an occlusion formed in a body organ or pathway is effectively utilized for fracturing the occluding matter and for perforating a passage thereinside, that may allow crossing and/or removing the occluding matter.

The balloon catheter of the invention is preferably constructed from concentric tubes having an inflatable member, such as a balloon, attached to their distal ends. The inflatable member can be a sleeve having tapering ends that can be sealably attached to the distal end portions of the inner and outer tubes of the catheter device, such that the lumen obtained between the inner and outer tubes can be used as an inflation lumen.

The manner in which the distal tapered extremity of the balloon is affixed to the distal end of the flexible inner catheter shaft permits the distal end of said balloon to roll and expand in response to increased pressure inside the catheter system. Similarly, as a result of this pressure increase, the inner shaft is caused to be stretched distally. Subsequently, when the pressure inside the catheter system is reduced, the elasticity of the inner shaft causes retraction (i.e. in a proximal direction) of the inner shaft tip to its original position in response to decreased pressure. A rapid, reciprocating pressure cycle (having a frequency in the sonic or subsonic range) thus causes a correspondingly rapid linear oscillatory motion of the distal tip of the guide wire which has been immobilized within the lumen of the inner catheter shaft. In this way, the rapid cyclical distal-proximal movement of the guide wire tip, together with the shock waves set up within the volume of blood situated between the inner shaft tip and the obstruction, may be used to progressively cut through an intravascular lesion located in the region of the distal end of the guide wire.

As mentioned hereinabove, the ability of the distal end of the balloon to roll and expand in response to increased pressure inside the catheter system is determined by the manner in which said distal end is affixed to the inner shaft. Essentially, the distal end of the balloon needs to be attached to the inner shaft in such a way that, during the part of the examplary method of use wherein said balloon is caused to oscillate, said distal end is intussuscepted. This may be achieved in two different ways:

### I. Pre-charged balloon conformation

In this conformation, the balloon is attached to the distal end of the inner shaft during manufacture such that its distal end is always intussuscepted (i.e. inwardly-folded onto the catheter shaft. This conformation may be achieved in a number of different ways as will be discussed further hereinbelow.

### II. Non-charged balloon conformation

In this conformation, the distal end of the balloon is attached to the inner shaft of the catheter in a conventional, non-intussuscepted manner. The distal intussusception is then created by the operator by means of moving the inner shaft in a proximal direction (in relation to the outer shaft). The inner shaft is then locked in place, thereby preserving the distal intussusception created by this procedure.

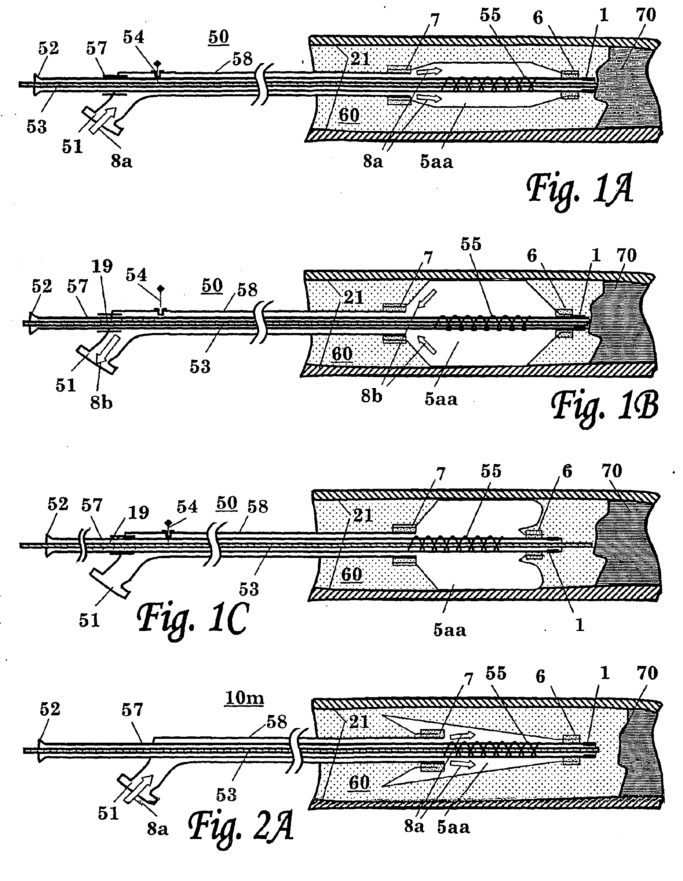

The balloon catheter of the present invention may be constructed as an over-the-wire catheter (e.g. as shown in Figs. 1A to 1C and 2A to 2B) or as a single-operator (i.e. rapid exchange) catheter. The vibration mechanism in latter implementation is substantially similar to the mechanism described hereinbelow with reference to the over-the-wire configurations shown in Figs. 1A to 1C. The rapid exchange catheter's structure mainly differs in that the lumen of its inner shaft may be accessed via a lateral port provided between the proximal and distal ends of the catheter. Such catheter configurations are well known in the art and described in many patent documents including co-owned, copending international patent application no. PCT/IB2006/002958 (published as WO 2007/042936).

In addition, the catheter may also be manufactured using bi-lumen catheter tubing (for at least a portion of the total length of the catheter), as will be described hereinbelow.

In a preferred embodiment, the aforementioned balloon catheter is manufactured as a sterile, single use catheter, which is entirely disposable.

The balloon catheter is, as mentioned hereinabove, connected to a reusable pressure generator console, wherein said console comprises a pressure pump, a pressure adjustment interface and a display providing control information for the physician. In one embodiment, the pressure generator console includes a piston and a chamber with an actuation member attached to the piston. The chamber may be used to introduce inflation fluid (e.g. contrast material or saline solution) into the pressure generator and the inflation lumen. A pressure sensor/gauge and a balloon sizing scale may be incorporated into the catheter assembly to assist the treating physician in monitoring the procedure. A longitudinally oscillating drive, such as a solenoid and/or a rotary electrical motor, may be operatively connected to the pressure generator.

The balloon catheter device of the invention can be introduced into the body of the treated subject via an incision, and advanced therethrough over the guidewire to the treatment site, as carried out in conventional catheterization procedures. Radiopaque markers provided on the catheter device (and/or on the guidewire it is threaded on), or any other suitable visioning technique, may be used to guide the balloon catheter device to the treatment site. After reaching the treatment site the inflatable member is inflated with a suitable inflation fluid to anchor and center the catheter device thereinside, such that a volume of fluid (e.g., blood) is delimited by said inflatable member and the proximal face of said occluding matter.

The inflatable member preferably has an expandable distal end portion designed to distally expand in response to pressure increments provided therein, and the inner tube (or a portion thereof) is preferably made resilient to allow distal elongation thereof. Repeated distal expansions of the inflatable member may be used to cause the inner tube to repeatedly stretch and retract axially in alternating (distal and proximal) directions. The inner tube of the balloon catheter is designed to grasp the guidewire passing in it prior to such distal expansions such that the alternating axial movements of the inner tube are transferred to the guidewire which is advantageously used to fracture the occluding matter by repeatedly ramming into it.

Figs. 1A to 1C demonstrate one possible procedure for catheter deployment in front of the occlusion **70** using the balloon catheter of the invention **50.** In this example non-charged balloon **5aa** of balloon catheter **50** has proximal and distal tapering ends attached to the outer surface of a distal portion of outer shaft **58** and of inner shaft **57,** at attachment points **7** and **6,** respectively. Catheter **50** may be advanced towards the treatment site over guide wire **53** threaded through the lumen of inner shaft **57.** Catheter **50** should be placed as near as possible to occlusion **70,** preferably such that its distal tip **1** contacts said occlusion. Once catheter **50** is placed in the treatment site balloon **5aa** may be inflated by introducing pressurized inflation media (e.g., fluid, designated by arrows **8a**) via inflation fluid port **51.** The inflation media passed via inflation fluid lumen defined between inner wall of outer shaft **58** and the outer surface of inner shaft **57.** In its inflated state (Fig. 1B) lateral sides of balloon **5aa** are pressed against the inner wall **21,** of vessel **60,** thereby anchoring it thereto.

After anchoring the balloon in the treatment site the physician may manipulate the inflated balloon by releasing a grasping element **54** (immobilizer), thus allowing inner shaft **57** to be moved proximally relative to outer shaft **58.** Inner shaft **57** is retracted proximally and anchored at its new location by restoring the grasp thereof by grasping element **54** (Fig. 1C). Graduated scale **19,** provided on a proximal portion of inner shaft **57,** may be used to assist the operator in determining the length of inner shaft **57** which has been retracted. Proximal retraction of inner shaft folds the distal end of balloon **5aa** proximally inwardly and shortens the balloon's length and consequently reduces its inflated volume as portions of inflation fluid are discharged therefrom (designated by arrows **8b**).

The discharged portions of inflation fluid may be received by an inflation fluid reservoir (not shown) via inflation fluid port 51 or via a dedicated discharge outlet (not shown).

At this state distal end **1** of inner shaft **57** may be vibrated about its longitudinal axis by applying an oscillator pressure source for periodically changing the pressure of the inflation media in balloon **5aa.** Such periodical pressure changes cause corresponding lengthening and shortening of the lengths of balloon **5aa** and resilient inner shaft **57** (or resilient intermediate portions thereof **55**) which may be employed for rupturing the occlusion and thereby opening a passage pathway therethrough.

The operator may advance guidewire **53** such that distal end portions thereof may leave inner shaft **57** through its distal end opening, e.g., such that 1 to 5 mm of the wire protrudes from the distal end of the catheter, as demonstrated in Fig. 1C. This is preferably carried out by advancing proximal portions of guidewire **53** distally through proximal end opening **52** of inner shaft **57** such that a distal portion thereof protrudes outwardly via the proximal end opening (at distal tip **1)** of inner shaft **57.**

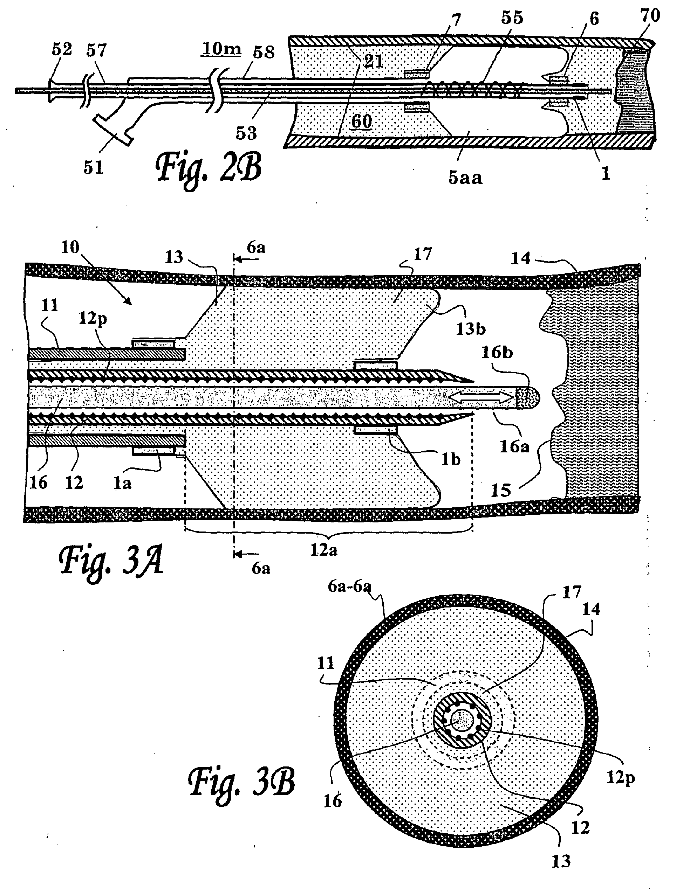

Figs. 2A and 2B demonstrate another possible procedure for catheter deployment in front of the occlusion. In this example a modified balloon catheter **10m** is used, wherein inner shaft **57** is affixed to outer shaft **58** (e.g., using a suitable adhesive), and balloon **5aa** is folded proximally (backwardly) thus forming an arrow-like shape which tapers towards its distal attachment point **6,** as shown in Fig. 2A. This folded state may be retained by folding the balloon into this folded state under heat and/or pressure (e.g., while folding the balloon in the manufacturing process the balloon will maintain its shape if the "wings" of the folded jacket will remain tight).

Catheter **10m** may be advanced towards the treatment site over guide wire **53** threaded through the lumen of inner shaft **57.** Catheter **10m** is preferably placed adjacent to occlusion **70,** preferably such that its distal tip **1** contacts said occlusion. Once catheter **10m** is placed in the treatment site balloon **5aa** may be inflated by introducing pressurized inflation media (designated by arrows **8a**) via inflation port **51.** Inflation media is passed via inflation fluid lumen defined between inner wall of outer shaft **58** and the outer surface of inner shaft **57.** In its inflated state (Fig. 2B) lateral sides of the backwardly folded balloon **5aa** are pressed against the inner wall **21** of vessel **60,** thereby anchoring it thereto. Due to its initial folded state the distal end of the inflated balloon gains a rounded shape, as shown in Fig. 2B.

In this state distal end **1** of inner shaft **57** may be vibrated about its longitudinal axis by applying an oscillatory pressure source for periodically changing the pressure of the inflation media in balloon **5aa.** Such periodical pressure changes cause corresponding lengthening and shortening of the lengths of balloon **5aa** and resilient inner shaft **57** (or resilient intermediate portions thereof **55)** which may be employed for rupturing the occlusion and thereby opening a passage pathway therethrough.

The operator may advance guidewire **53** such that distal end portions thereof may leave inner shaft **57** through its distil end opening, e.g., such that 1 to 5 mm of the wire protrudes from the distal end of the catheter, as demonstrated in Fig. 2B. This is preferably carried out by advancing proximal portions of guidewire **53** distally through proximal end opening **52** of inner shaft **57** such that a distal portion thereof protrudes outwardly via the proximal end opening (at distal tip **1**) of inner shaft **57.**

Figs. 3A and 3B schematically illustrates a preferred embodiment of the balloon catheter device **10** of the invention operatively situated in an occluded body passageway **14** (e.g., blood vessel) comprising occluding matter **15.** Balloon catheter **10** comprises an inflatable member **13** attached in a proximal attachment **1a** to a distal end portion of outer tube **11** of balloon catheter device **10,** and in a distal attachment **1b** to a distal end portion of inner, tube **12** passing in outer tube **11.**

Inflatable member **13** is preferably made from a non-compliant or semi-compliant sleeve having tapering extremities that are adapted to fit over the outer surfaces of outer and inner tubes, **11** and **12.** Inflatable member **13** is configured to perform radial expansion, when filled with a suitable inflation media **17,** and thereafter distal expansion of its distal portion **13b,** when said inflation media **17** is pressurized. As demonstrated in the longitudinal and cross-sectional views of the catheter device **10** shown in Figs. 3A and 3B, radial expansion of inflatable member **13** presses its lateral wall against the inner side of the body pathway or organ **14** in which it is placed and thereby centers and anchors catheter device **10** in place.

Inner shaft tube **12** can be affixed to the outer shaft tube **11,** or it may be reversibly attached to it via releasable locking means (not shown) provided at a proximal portion thereof, such that a distal portion **12a** of inner tube **12** protrudes outwardly via the distal end opening of outer tube **11.** At least a portion of inner tube **12** is made resilient to allow distal elongation thereof in response to, distal expansions of inflatable member **13.** For example, inner tube **12,** or a portion thereof, may be manufactured from a resilient material (e.g., Pebax and/or Nylon Blend), or from a soft and flexible material comprising a resilient element such as a spring. Various ways of making a section of the inner tube resilient are described in US application No. 60/726,180 and in international patent application no. PCT/IB2006/002958 (published as WO 2007/042936) of the same applicant hereof.

In the preferred embodiment of the invention inflatable member 13 is made from a non-compliant or a semi-compliant sleeve having tapering ends designed to fit over the outer surfaces of inner tube **12** and outer tube **11.** The inner surface of the proximal end of the flexible sleeve is fitted and attached on the outer surface of a distal end portion of outer tube **11** at proximal attachment **1a**, and the outer surface of the distal end of the flexible sleeve is fitted and attached on the oxter surface of a distal, end portion of outer tube **11** at distal attachment **1b**.

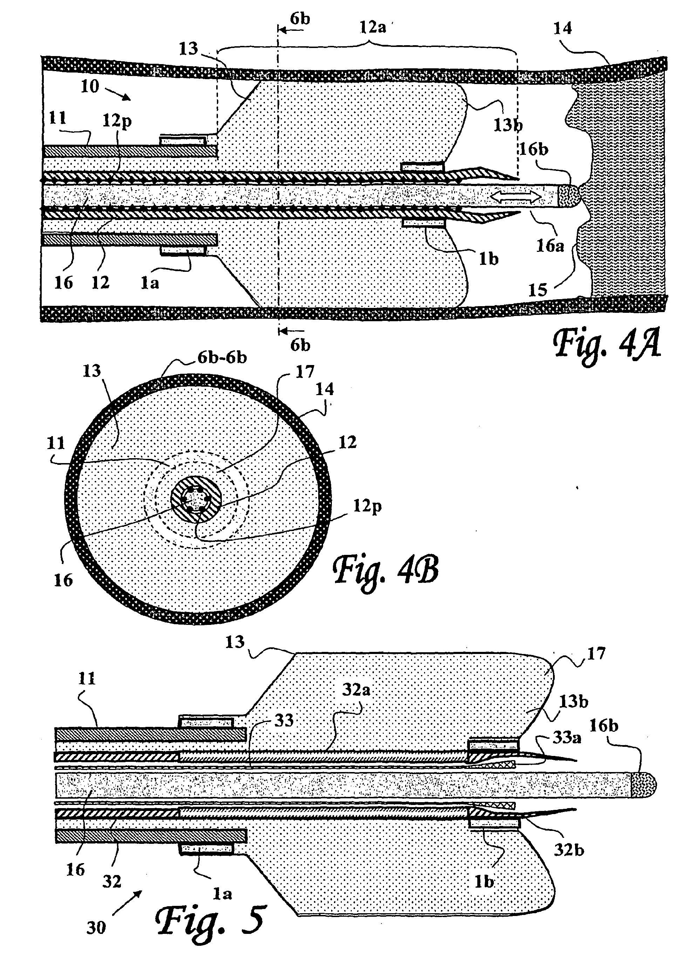

The location of distal attachment **1b** on, distal portion **12a** of inner tube **12** is chosen such that distal end portions of inflatable member **13** are folded proximally inwardly over a distal end portion of inner tube **12.** In this way distal expansion of distal portion **13b** of inflatable member **13** is achieved by increasing the pressure of the inflation media **17** inside inflatable member **13** which in response force the inwardly folded distal portions of inflatable member **13** to unfold distally and restore the original shape of inflatable member **13,** thereby increasing the volume of inflatable member **13** and stretching distal portions **12a** of outer tube **12** distally, as demonstrated in Fig. **4A****.**

**A** ramming tool **16** (e.g., guidewire) passing in the lumen of inner tube **12,** and mechanically coupled thereto, is used for the fracturing and/or tunnelling of occlusion **15,** as shown in Figs. 4A and 4B. The mechanical coupling between ramming tool **16** and inner tube **12** may be achieved by making distal portion of inner tube **12** from a flexible material capable of being pressed over, and thereby retain, a. portion of ramming tool **16** passing thereinside.

The inner surface of inner tube **12** may be roughened in order to increase its friction constant for enhancing the gripping forces that may be applied by it upon ramming tool **16.** For example, the roughening to the inner surface of inner tube **12** may be obtained by forming (or attaching) protrusions **12p** thereon (e.g., by a chemical process, such as chemical deposit of particles on the exposed inner wall of the tube).

Outer tube **11** may be manufactured by extrusion from a polymeric material, such as Nylon, preferably from Polyurethane. The inner diameter of outer tube **11** may generally be in the range of 0.4 to 1.0 mm, preferably about 0.75 mm, and its length may generally be in the range of 1000 to 2000 mm, preferably about 1500 mm.

Inner tube **12** may be manufactured by extrusion from a metallic material, such as stainless steel, preferably from SS 316, to which a resilient distal end portion tube may be attached, as will be exemplified herein later. The inner diameter of Proximal inner tube **12** may generally be in the range of 0.2 to 0.8 mm, preferably about 0.4 mm, and its length may generally be in the range of 1000 to 2000 mm, preferably about 1450 mm. The length of distal portion **12a** of inner tube **12** protruding outwardly via the distal end opening of outer tube **11** is generally in the range of 5 to 30 mm, preferably about 10 mm.

In a preferred embodiment of the invention the length of distal portion **12a** of inner tube **12** may be adjusted by the operator via releasable immobilizing means (not shown) provided at a proximal end portion of the device, that allows the operator to move inner tube **12** distally/proximally and affix it to outer tube **11** at a desired location. In such implementation the operation of the balloon catheter may be divided to a number of stages in which the length of distal portion **12a** of inner tube **12** is gradually increased according to the progress of the perforation (or tunneling) performed in occlusion **15.**

Inflatable member **13** may be manufactured by blowing from a polymeric material, such as Nylon, preferably from Nylon-Pebax blend. The diameter of inflatable member **13** may generally be in the range of 1.5 to 8 mm, preferably about 3 mm, and its length may generally be in the range of 10 to 50 mm, preferably about 20 mm. Attachment of inflatable member **13** to inner and outer tubes **12** and **11,** at distal and proximal attachments **1b** and **1a**, respectively, may be achieved by means of bonding, preferably by thermal bonding process.

Inflation media **17** may be any type of conventional inflation media used in balloon catheters. For example, a type of Saline or contrast materials. The pressure in inflatable member **13** when first inflated to anchor catheter device **10** in place, is generally in the range of 1 to 6 atmospheres. When the inflation media **17** in inflatable member **13** is further pressurized, for effecting distal expansions of distal portion **13b,** the inflatable media **17** is further pressurized to a pressure generally in the range of **1** to **10** atmospheres. The time intervals in which the inflation media **17** is repeatedly pressurized for effecting said distal expansions of distal portion **13b** may be varied according to the type of occlusion to be opened. For example, in a specific embodiment of the invention the pressure of the inflation fluid **17** may be periodically changed between 6 to 8 atmospheres in frequencies in the range of 1 to 20 Hz, preferably about 10 Hz.

The elongation of inner tube **12** may generally be in the range of 0.5 to 3 mm, preferably about 1 mm. The diameter of ramming tool/guidewire 16 may generally be in the range of 0.23 mm to 0.89 mm (0.009 inch to 0.035 inch), preferably about 0.36 mm (0.014 inch), and its length may generally be in the range of 180 to 250 mm, preferably about 190 mm.

Fig. 5 illustrates an implementation of a balloon catheter 30 wherein the catheter comprises coupling means for strengthening the grip of the distal portion of the inner tube over ramming tool **16.** The coupling means comprise pull member **33** disposed in inner tube **32** along partial (or entire) length thereof. The distal end of pull member **33** preferably comprises wedge shaped locking member **33a.** The distal end section **32b** of inner tube **32** may be configured accordingly to comprise said wedge shaped locking member **33a** thereinside, as shown in. Fig. 5. Namely, the inner diameter of said distal end section **32b** is greater near the distal tip and it is gradually decreased towards the proximal end of the distal end section **32b.**

The coupling means implemented by pull member **33** enhances the grip of inner tube **32** over ramming tool **16,** particularly during distale expansions of inflatable member **13,** as occurring during repeated distal expansions of inflatable member **13.** During said distal expansions the pressure in inflatable member **13** is increased which in turn stretches flexible section **32a** distally. Distil stretch of flexible section **32a** cause in turn distal movement of distal end section **32b** of inner tube **32** which locks wedge shaped locking member **33a** due to its tapering inner shape, and thus forces wedge shaped locking member **33a** to grip ramming tool **16.**

Flexible section **32a** can be made of resilient material, such as Pebax, and it may be embedded into a distal portion of inner tube **32,** by thermal bonding. The length of flexible section **32a** may generally be in the range of 10 to 100 mm, preferably about 80 mm. Wedge shaped locking member **33a** can be made of a metallic material, such as stainless steel, and it may be combined or installed in inner tube **32** by adhesives. The length of wedge shaped locking member **33a** may generally be in the range of 1 to 3 mm, preferably about 2 mm, and its diameter should be slightly greater than the diameter of ramming tool **16.**

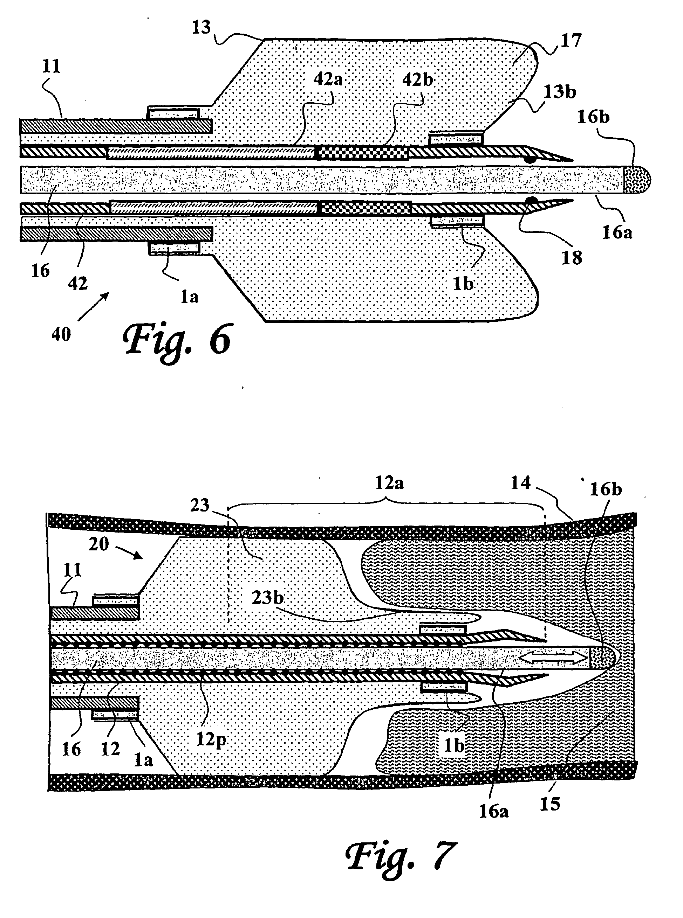

Fig. 6 illustrates an implementation of a balloon catheter **40** wherein the inner tube **42** comprises a resilient section **42a** in which coupling means **42b** are provided for establishing a grip over ramming tool **16.** Resilient section **42a** is configured to affect lengthening and retraction of the distal end section of the inner tube **42** during pressure increments in inflation media **17.** Coupling means **42b** is preferably made from a soft and flexible material (e.g., Elastomerics) embedded in the distal end section of resilient section **42a** for gripping ramming tool **16** during pressure increments in inflation media **17.**

In one example, the operation of the balloon catheter of the invention comprises the steps of:
i) inserting the distal end of a pre-charged balloon catheter **40** into the treatment site such that its distal tip is placed in the vicinity ,(e.g., 1 to 5 mm) of the occluding matter **15;**
ii) inflating the inflatable member **13** with a suitable inflation media **17** pressurized to about 1 atmospheres to anchor and center the distal end section of the balloon catheter in place;
iii) optionally, manually ramming the distal tip **16b** of the ramming tool **16** into the occluding matter, by pulling and pushing it at its proximal end, and if such manual operation is not sufficient for passing the occlusion;
iv) advancing ramming tool **16** distally such that a distal end portion thereof .(e.g., about 1-5 mm) protrudes distally via the distal end opening of inner tube;
v) pressurizing the inflation media **17** to about 2 atmospheres which in turn causes distal expansion of the distal section **13b** of the inflatable element **13** thereby causing stretching and lengthening of coupling means **42b** which in turn results in it being tightly pressed over ramming tool **16.** In this way the stretching and lengthening of coupling means **42b** is utilized to press its wall over ramming tool **16,** thereby gripping said tool;
vi) pressurizing the inflation media **17** to about 4 atmospheres which in turn causes distal expansion of the distal section **13b** of the inflatable element **13,** which in turn causes stretching and lengthening of resilient section **42a,** and moves the distal end section of ramming **16** distally such that its distal tip **16b** is rammed into the occluding matter **15;**
vii) reducing the pressure of the inflation media **17** back to about 2 atmospheres which cause resilient section **42a** to restore its un-stretched length and inwardly fold back the distal section **13b** of inflatable element **13,** while maintaining tight grip of coupling means **42b** over ramming tool **16** and thereby retracting it proximally;
   and
viii) repeating steps vi) and vii) until the desired perforation of the occluding material is achieved;
ix) advancing the modified guidewire distally until it passes the occluding matter and thereby providing a passage therethrough; and
x) carrying out the conventional treatment suitable for opening the occlusion (e.g., balloon inflation, stenting, and any other technique well known to the skilled artisan).

In a modification of the basic operating procedure described immediately hereinabove, the pressure levels of the inflation media may be manipulated such that a "ratchet mechanism" is created, thereby automatically advancing the wire inside the occlusive material. This is achieved by means of cyclically reducing the pressure to below the grasping pressure reached in step (v) of the procedure described above (e.g. to below a level of about 2 atmospheres), and then elevating the pressure to a level above said grasping pressure. In this way, the guide wire is released from being grasped by the inner shaft (when the pressure is lowered to beneath, grasping pressure), and then re-grasped at a more proximal point along its length, thereby advancing the distal tip of the wire towards or inside the occluding material.

The advantage of this modified mechanism is the automatic advancement of the guide wire thereby obviating the need for manually reducing the pressure inside the balloon in order to release the grasp, and then manually advancing the guide wire and repeating the grasping phase (step v) at a more proximal location, followed by the stretching phase (step vi).

In the case of balloon catheters utilizing non-charged balloons, the above-described procedure may be employed with the addition of the following step: after anchoring of the balloon at the treatment site (step (ii)), the operator may manipulate the inflated balloon by releasing a grasping element (immobilizer), thus allowing the inner shaft to be moved proximally relative to the outer shaft. The inner shaft is then retracted proximally and anchored at its new location by re-applying the grasping element. This step is described in more detail hereinabove in relation to the device exemplified in Fig. 1C.

Optionally, following step viii) and prior to step ix), the following steps may be performed:
A) stopping the repeated pressure pulses (steps vi and vii), reducing the pressure of the inflation media **17** to about 1 atmospheres and releasing inner tube immobilizer to increase the length of distal portion **12a** in order to change the state of inflatable member into a second folded state in which a smaller portion of the length of inflatable member **13** is folded proximally inwardly;
B) restoring grasping pressure of inflation media in inflatable member, restoring immobilization of the inner tube, and applying the repeated pressure pulses (steps vi, and vii) using similar frequencies within a similar period of time;
C) repeating steps A) and B) to apply the repeated pressure pulses in a third folded state (e.g., smaller length of inflatable member **13** is folded proximally inwardly); Resilient section **42a** may be manufactured by extrusion from a polymeric material, such as Nylon blend, preferably from Pebax-Nylon blend. The length of resilient section **42a** may generally be in the range of 10 to 100 mm, preferably about 80 mm, and it may be attached to inner tube **42** (e.g., thermal bonding and/or Induction bonding).

Coupling means **42b** may be implemented using a soft material such as silicone or polymer, and/or by embedding a braided section in flexible section **42b.** The grip applied by coupling means **42b** may be further enhanced by coating its inner surface with friction enhancing material, such as Silicon coating, by embedding an inner silicone tube segment or a coil in resilient section **42b.** Additionally or alternatively, coupling means **42b** may have a rectangular cross-sectional shape in order to increase buckling thereof, and thus enhance its grip, when it is pressed against ramming tool **16.**

The coupling means may further comprise gripping protrusions **18** attached to, or formed on, the inner wall of inner tube **42,** near its distal tip. Gripping protrusions **18** are configured to be in contact with the surface **(16a)** of ramming tool **16** and thus grip its, distal end section during elongation and retraction of inner tube **42a.** Gripping protrusions **18** are configured to allow enhanced pushing/pulling forces exerted from the proximal end of ramming tool **16** to overcome the friction forces between gripping protrusions **18** and ramming tool **16** in order to permit re-positioning and advancing ramming tool **16** distally such that a distal end portion thereof protrudes distally via the distal end opening of inner tube.

Fig. 7 illustrates a preferred embodiment of a balloon catheter **20** of the invention wherein a distal end portion **23b** of inflatable member 23 is, made narrow. The structure and principal of operation of balloon catheter **20** are substantially similar to those of balloon catheter **10** described with reference to Figs. 3A, 3B, 4A and 4B. However, due to its narrow distal end section **23b,** inflatable member **23** of balloon catheter **20** may be advanced into perforated portions of occlusion **15,** as demonstrated in Fig. 7.

The diameter of inflatable member **23** may generally be in the range of 1.5 to 6 mm, preferably about 3 mm, and its length in the range of 10 to 50 mm. The diameter of narrow distal end section **23b** of inflatable member **23** may generally be in the range of 1 to 3 mm, preferably about 1 mm, and its length in the range of 5 to 20 mm.

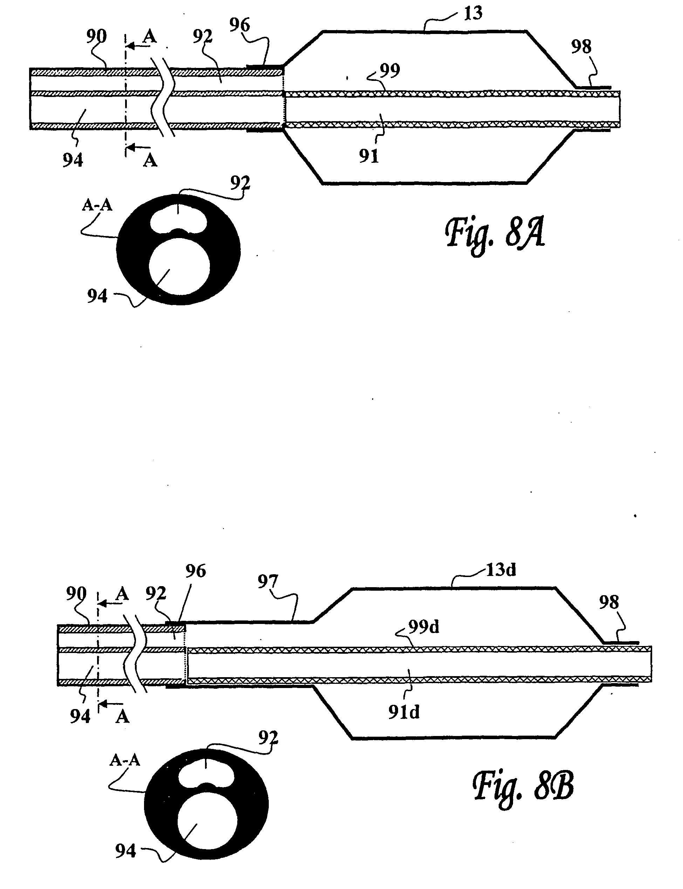

In another example, as mentioned hereinabove, the balloon catheter of the present invention may be constructed as a bilumen catheter. Fig. 8A illustrates one embodiment of the invention utilizing bi-lumen catheter tubing along at least a portion of the overall catheter length. In this figure (guidewire not shown), the proximal end of balloon **13** is attached to the external surface of bi-lumen conduit **90** at proximal attachment point **96,** said bi-lumen conduit comprising two parallel lumens: inflation fluid lumen **92** and guidewire lumen **94.** A cross-sectional view of the bi-lumen conduit taken at level A-A that shows the relative arrangement of the two lumens is provided in the lower part of this figure. While inflation fluid lumen **92** ends at the proximal balloon attachment point **96,** guidewire lumen **94** continues beyond the proximal attachment point **96** of balloon **13,** said lumen becoming continuous with the guidewire lumen **91** of distal conduit **99.** The outer surface of said distal conduit, which contains an elastically deformable region, provides a distal balloon attachment point **98.**

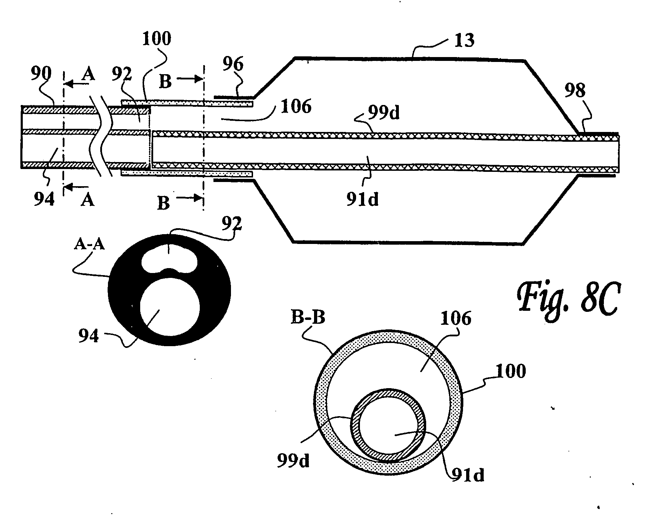

In certain circumstances, it is desirable to provide a bi-lumen catheter of the type described immediately hereinabove, in which the length of the elastically-deformable distal conduit is not limited by the length of the balloon. Figures 8B and 8C illustrate to further examples utilizing a bi-lumen conduit, in which this length restriction is removed.

Thus, in the example of the catheter shown in Fig. 8B, the modified balloon, **13d,** has an elongated proximal neck, **97**. The increase in length of the balloon in this example permits the use of a longer distal conduit **99d.** All the other elements in this example are the same as those shown in Fig. 8A.

Fig. 8C illustrates another example of the bi-lumen configuration described hereinabove. In this case, the catheter further comprises a connecting tube segment **100** positioned between the conduit proximal portion (i.e. bi-lumen conduit) **90** and the proximal attachment point of the balloon **96.** Said connecting tube segment, shown in cross-sectional view in the lower right portion of Fig. 8C, contains two concentrically arranged conduits: an outer conduit having a lumen **106** that is in fluid communication with the fluid passage lumen **92** of the proximal bi-lumen conduit **90,** and an inner conduit formed by the elastic section-containing distal conduit **99d,** the lumen **91d** of which is in fluid communication with the guidewire lumen **94** of said bi-lumen conduit **90.** As seen in the figure, the presence of connecting tube segment **100** permits the use of a longer distal conduit **99d** than is possible in the example depicted in Fig. 8A.

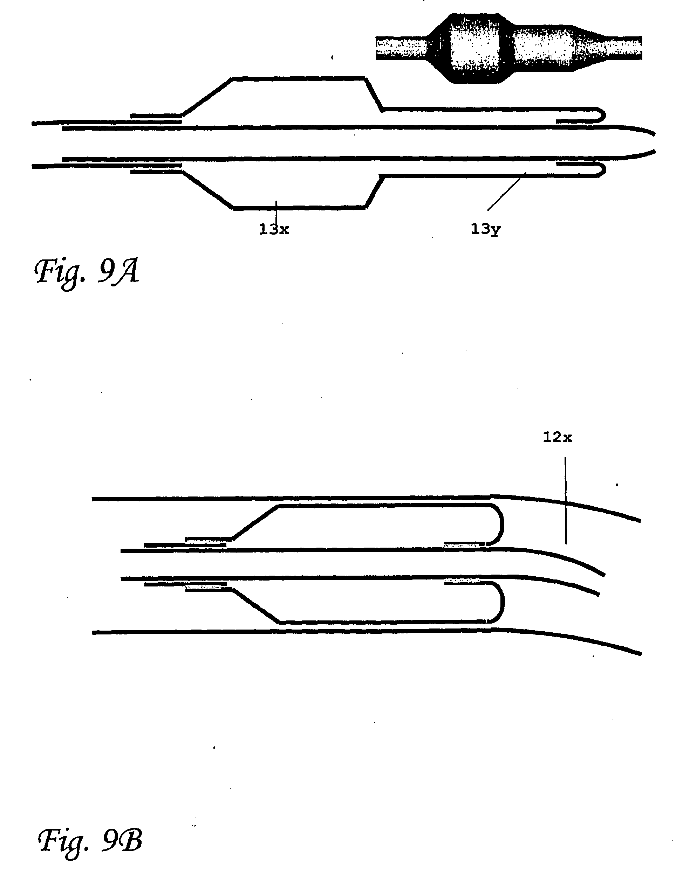

Fig. 9A depicts an alternative embodiment of balloon **13,** which may be advantageously used when the balloon catheter needs to negotiate a curve or bend in the blood vessel which is being treated. This embodiment of the balloon is constructed such that it possesses a stepped shape, having a broader proximal portion **13x** and a narrower distal portion **13y.** As the balloon is advanced towards the curved region of the blood vessel, the narrow distal balloon portion **13y** enabling the catheter and guide wire tips to be centered within said curved or arched region. This balloon design may also be used as a deflectable catheter, wherein the narrow distal portion **13y** is manually diverted towards the occlusion, centering the tip with respect to vessel geometry.

Fig. 9B depicts a further, alternative embodiment that may also be used to negotiate curved or arched regions of the vasculature. In this embodiment, the distal end of the inner shaft of the catheter beyond the distal end of the balloon **12x** is considerably longer than in the embodiments hereto described, thereby permitting manually diversion of this extension around the arched portion of the blood vessel.

All of the abovementioned parameters are given by way of example only, and may be changed in accordance with the differing requirements of the various embodiments of the present invention. Thus, the abovementioned parameters should not be construed as limiting the scope of the present invention in any way. In addition, it is to be appreciated that the different tubes, sleeves, balloons, and other members, described hereinabove may be constructed in different shapes (e.g. having oval, square etc. form in plan view) and sizes from those exemplified in the preceding description.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention as defined in the claims.

## Claims

1. A balloon catheter (50) comprising:
an inner shaft (57) disposed within an outer shaft (58) such that the distal end of the inner shaft (57) extends beyond the distal end of the outer shaft (58), wherein the lumen of said inner shaft (57) is suitable for allowing the passage of a guidewire (53) through all or part of its length;
a balloon (5aa) attached at its proximal end to said outer shaft (58) and at its distal end to said inner shaft (57); and
means for the introduction of an expansion fluid (8a) into the annular space formed between the inner surface of the outer shaft (58) and the outer surface of the inner shaft (57) and therefrom into the lumen of said balloon (5aa), and for the removal thereof;
wherein the inner shaft (57) is constructed such that following radial expansion of the balloon (5aa) to a first expanded state, a portion of said inner shaft (57) is capable of responding to further longitudinal expansion of the balloon (5aa) to a second expanded state by reducing its inner diameter thereby being able to grasp a guidewire (53) placed within the lumen of said inner shaft (57), thereby preventing movement of said guidewire (53) relative to said inner shaft (57), **characterised in that**
said inner shaft (57) is capable of responding to further expansion of the balloon (5aa) from said second expanded state to a third expanded state by increasing its length from a resting value, and of responding to subsequent partial deflation back to said second expanded state by reducing its length back to said resting value.

2. The balloon catheter according to claim 1, wherein the inner shaft comprises at least a first elastically deformable portion and second elastically deformable portion, wherein, following radial expansion of the balloon to a first expanded state,
said first elastically, deformable portion of the shaft is capable of responding to further longitudinal expansion of the balloon to a second expanded state by reducing its inner diameter thereby being able to grasp a guidewire placed within the lumen of said inner shaft and thereby preventing movement of said guidewire relative to said inner shaft, and wherein said second elastically deformable portion of the shaft is capable of responding to further longitudinal expansion of the balloon from said second expanded state to a third expanded state by increasing its length from a resting value, and of responding to subsequent partial deflation back to said second expanded state by reducing its length back to said resting value.

3. The balloon catheter according to claim 1, wherein the distance between the points of attachment of the balloon to the inner and outer shafts is less than the overall length of the balloon when said balloon is in the first expanded state.

4. The balloon catheter according to claim 1, wherein the proximally-located and distally-located elastically deformable portions are each constructed of blends of nylon and Pebax.

5. The balloon catheter according to 1, further comprising supplementary guide wire grasping means.

6. The balloon catheter according to claim 5, wherein the supplementary guide wire grasping means comprise protrusions on the inner wall of the inner shaft.

7. The balloon catheter according, to claim 5, wherein the supplementary guide wire grasping means comprise wedge-shaped locking means.

8. The balloon catheter according to claim 1, wherein the balloon is attached at its distal end in an inverted manner, such that the external surface of said balloon is in contact with, and attached to, the outer surface of the inner shaft.

9. The balloon catheter according to claim 1, wherein the balloon is attached at its distal end in a non-inverted manner, such that the internal surface of said balloon is in contact with, and attached to, the outer surface of the inner shaft.

10. The balloon catheter according to claim 1, wherein said catheter is constructed as an over-the-wire catheter.

11. The balloon catheter according to claim 1, wherein the inner shaft is capable of being moved along its longitudinal axis in relation to the outer shaft, and wherein said catheter further comprises means for immobilizing said inner shaft.

12. The balloon catheter according to claim 1, wherein said catheter is constructed as a rapid exchange catheter.

13. The balloon catheter according to claim 1, wherein the balloon is a stepped balloon, having a broader proximal portion and a narrower distal portion.

## Patentansprüche

1. Ballonkatheter (50), enthaltend:
einen Innenschaft (57), der in einem Außenschaft (58) derart angeordnet ist, dass sich das distale Ende des Innenschaftes (57) über das distale Ende des Außenschaftes (58) hinaus erstreckt, wobei sich das Lumen des Innenschaftes (57) dazu eignet, den Durchgang eines Führungsdrahtes (53) durch die gesamte oder einen Teil seiner Länge zu gestatten;
einen Ballon (5aa), der an seinem proximalen Ende an dem Außenschaft (58) und an seinem distalen Ende an dem Innenschaft (57) angebracht ist; und Einrichtungen für die Einleitung eines Ausdehnungsfluids (8a) in den ringförmigen Zwischenraum, der zwischen der Innenoberfläche des Außenschaftes (58) und der Außenoberfläche des Innenschaftes (57) ausgebildet ist, und von dort in das Lumen des Ballons (5aa) und für eine Ausleitung desselben;
wobei der Innenschaft (57) derart ausgebildet ist, dass nach der radialen Ausdehnung des Ballons (5aa) in einen ersten ausgedehnten Zustand, ein Abschnitt des Innenschaftes (57) in der Lage ist, auf eine weitere Längsausdehnung des Ballons (5aa) in einen zweiten ausgedehnten Zustand zu reagieren, indem dessen Innendurchmesser verringert wird, wodurch er in der Lage ist, einen Führungsdraht (53) zu greifen, der innerhalb des Lumens des Innenschaftes (57) angeordnet ist, wodurch eine Bewegung des Führungsdrahtes (53) im Bezug auf den Innenschaft (57) verhindert wird, **dadurch gekennzeichnet, dass**
der Innenschaft (57) in der Lage ist, auf eine weitere Ausdehnung des Ballons (5aa) von seinem zweiten ausgedehnten Zustand in einen dritten ausgedehnten Zustand zu reagieren, indem dessen Länge von einem Ruhewert erhöht wird, und auf eine anschließende Teilentleerung zurück in den zweiten ausgedehnten Zustand durch Verringerung seiner Länge zurück in den Ruhewert zu reagieren.

2. Ballonkatheter nach Anspruch 1, bei dem der Innenschaft wenigstens einen ersten elastisch verformbaren Abschnitt und einen zweiten elastisch verformbaren Abschnitt enthält, wobei nach der radialen Ausdehnung des Ballons in einen ersten ausgedehnten Zustand
der erste elastisch verformbare Abschnitt des Schaftes in der Lage ist, auf eine weitere längliche Ausdehnung des Ballons in einen zweiten ausgedehnten Zustand zu reagieren, indem er seinen Innendurchmesser verringert, wodurch er in der Lage ist, einen Führungsdraht zu greifen, der in dem Lumen des Innenschaftes angeordnet ist, und **dadurch** eine Bewegung des Führungsdrahtes relativ zu dem Innenschaft verhindert, wobei der zweite elastisch verformbare Abschnitt des Schaftes in der Lage ist, auf eine weitere längliche Ausdehnung des Ballons von seinem zweiten ausgedehnten Zustand in einen dritten ausgedehnten zustand zu reagieren, indem er seine Länge aus einem Ruhewert vergrößert, und auf eine anschließende Teilentleerung zurück in den zweiten ausgedehnten zustand zu reagieren, indem er seine Länge zurück auf den Ruhewert verringert.

3. Ballonkatheter nach Anspruch 1, bei dem der Abstand zwischen den Anbringungspunkten des Ballons an dem Innen- und Außenschaft geringer ist als die Gesamtlänge des Ballons, wenn sich der Ballon in dem ersten ausgedehnten Zustand befindet.

4. Ballonkatheter nach Anspruch 1, bei dem der proximal angeordnete und der distal angeordnete elastisch verformbare Abschnitt jeweils aus einem Gemisch von Nylon und Pebax aufgebaut sind.

5. Ballonkatheter nach Anspruch 1, weiterhin enthaltend zusätzliche Führungsdrahtgreifeinrichtungen.

6. Ballonkatheter nach Anspruch 5, bei dem die zusätzlichen Führungsdrahtgreifeinrichtungen Vorsprünge auf der Innenwand des Innenschaftes enthalten.

7. Ballonkatheter nach Anspruch 5, bei dem die zusätzlichen Führungsdrahtgreifeinrichtungen keilförmige Verriegelungseinrichtungen enthalten.

8. Ballonkatheter nach Anspruch 1, bei dem der Ballon an seinem distalen Ende in umgekehrter Weise angebracht ist, so dass die Außenoberfläche des Ballons mit der Außenoberfläche des Innenschaftes in Kontakt und an dieser angebracht ist.

9. Ballonkatheter nach Anspruch 1, bei dem der Ballon an seinem distalen Ende in einer nicht umgekehrten Weise derart angebracht ist, dass die Innenoberfläche des Ballons mit der Außenoberfläche des Innenschaftes in Kontakt und an dieser angebracht ist.

10. Ballonkatheter nach Anspruch 1, wobei der Katheter als ein über dem Draht ausgebildeter Katheter aufgebaut ist.

11. Ballonkatheter nach Anspruch 1, bei dem der Innenschaft in der Lage ist, entlang seiner Längsachse im Bezug auf den Außenschaft bewegt zu werden, wobei der Katheter weiterhin Einrichtungen zum Festsetzen des Innenschaftes enthält.

12. Ballonkatheter nach Anspruch 1, wobei der Katheter als Schnellwechselkatheter ausgebildet ist.

13. Ballonkatheter nach Anspruch 1, bei dem der Ballon ein abgestufter Ballon ist, der einen breiteren proximalen Abschnitt und einen schmaleren distalen Abschnitt hat.

## Revendications

1. Cathéter à ballonnet (50) comprenant :
un arbre interne (57) disposé dans un arbre externe (58), de sorte que l'extrémité distale de l'arbre interne (57) s'étende au-delà de l'extrémité distale de l'arbre externe (58), dans lequel la lumière dudit arbre interne (57) est adaptée pour permettre le passage d'un fil guide (53) à travers tout ou partie de sa longueur ;
un ballonnet (5aa) fixé à son extrémité proximale audit arbre externe (58) et à son extrémité distale audit arbre interne (57) ; et
des moyens permettant l'introduction d'un fluide de dilatation (8a) dans l'espace annulaire formé entre la surface interne de l'arbre externe (58) et la surface externe de l'arbre interne (57) et de là dans la lumière dudit ballonnet (5aa), et permettant également son enlèvement ;
dans lequel l'arbre interne (57) est construit de sorte que suivant la dilatation radiale du ballonnet (5aa) dans un premier état dilaté, une partie dudit arbre interne (57) est capable de répondre à une dilatation longitudinale ultérieure du ballonnet (5aa) dans un second état dilaté, en réduisant son diamètre interne, en pouvant ainsi saisir un fil guide (53) placé dans la lumière dudit arbre interne (57), en empêchant ainsi le mouvement dudit fil guide (53) relativement audit arbre interne (57), **caractérisé en ce que**
ledit arbre interne (57) est capable de répondre à une dilatation ultérieure du ballonnet (5aa) depuis ledit second état dilaté à un troisième état dilaté, en augmentant sa longueur par rapport à une valeur de repos, et de répondre à un dégonflement partiel successif jusqu'audit second état dilaté, en réduisant sa longueur de nouveau à ladite valeur de repos.

2. Cathéter à ballonnet selon la revendication 1, dans lequel l'arbre interne comprend au moins une première partie élastiquement déformable et une second partie élastiquement déformable, dans lequel, après une dilatation radiale du ballonnet dans un premier état dilaté,
ladite première partie élastiquement déformable de l'arbre est capable de répondre à une dilatation longitudinale ultérieure du ballonnet dans un second état dilaté en réduisant son diamètre interne, en pouvant ainsi saisir un fil guide placé dans la lumière dudit arbre interne et en empêchant ainsi le mouvement dudit fil guide relativement audit arbre interne, et dans lequel ladite seconde partie élastiquement déformable de l'arbre est capable de répondre à une dilatation longitudinale ultérieure du ballonnet dudit second état dilaté à un troisième état dilaté, en augmentant sa longueur par rapport à une valeur de repos, et de répondre à un dégonflement partiel consécutif dans ledit second état dilaté, en réduisant sa longueur de nouveau à ladite valeur de repos.

3. Cathéter à ballonnet selon la revendication 1, dans lequel la distance entre les points de fixation du ballonnet aux arbres interne et externe est inférieure à la longueur totale du ballonnet quand ledit ballonnet est dans le premier état dilaté.

4. Cathéter à ballonnet selon la revendication 1, dans lequel les parties élastiquement déformables situées de manière proximale et de manière distale sont chacune réalisées en mélanges de nylon et de Pebax.

5. Cathéter à ballonnet selon la revendication 1, comprenant en outre des moyens supplémentaires de saisie du fil guide.

6. Cathéter à ballonnet selon la revendication 5, dans lequel les moyens supplémentaires de saisie du fil guide comprennent des saillies sur la paroi interne de l'arbre interne.

7. Cathéter à ballonnet selon la revendication 5, dans lequel les moyens supplémentaires de saisie du fil guide comprennent des moyens de blocage en forme de coin.

8. Cathéter à ballonnet selon la revendication 1, dans lequel le ballonnet est fixé à son extrémité distale d'une manière inversée, de sorte que la surface extérieure dudit ballonnet soit en contact avec la surface extérieure dudit arbre interne et fixée à celle-ci.

9. Cathéter à ballonnet selon la revendication 1, dans lequel le ballonnet est fixé à son extrémité distale d'une manière non-inversée, de sorte que la surface intérieure dudit ballonnet soit en contact avec la surface extérieure dudit arbre interne et fixée à celle-ci.

10. Cathéter à ballonnet selon la revendication 1, dans lequel ledit cathéter est construit comme un cathéter sur le fil.

11. Cathéter à ballonnet selon la revendication 1, dans lequel l'arbre interne est capable d'être déplacé le long de son axe longitudinal relativement à l'arbre externe, et dans lequel ledit cathéter comprend en outre des moyens pour immobiliser ledit arbre interne.

12. Cathéter à ballonnet selon la revendication 1, dans lequel ledit cathéter est construit comme un cathéter à échange rapide.

13. Cathéter à ballonnet selon la revendication 1, dans lequel le ballonnet est un ballonnet étagé, ayant une partie proximale plus large et une partie distale plus étroite.
